(19) Europäisches Patentamt / European Patent Office / Office européen des brevets

(11) **EP 4 382 608 A1**

(12) **EUROPEAN PATENT APPLICATION**
published in accordance with Art. 153(4) EPC

(43) Date of publication:
**12.06.2024 Bulletin 2024/24**

(21) Application number: **22852921.0**

(22) Date of filing: **27.07.2022**

(51) International Patent Classification (IPC):
*C12N 15/53* (2006.01)    *C12N 1/15* (2006.01)
*C12N 1/19* (2006.01)    *C12N 1/21* (2006.01)
*C12N 5/10* (2006.01)    *C12N 9/04* (2006.01)
*C12P 21/02* (2006.01)    *C12Q 1/32* (2006.01)

(52) Cooperative Patent Classification (CPC):
**C12N 5/10; C12N 9/0004; C12N 15/74;
C12N 15/80; C12N 15/81; C12P 21/02; C12Q 1/32**

(86) International application number:
**PCT/JP2022/028959**

(87) International publication number:
**WO 2023/013498 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **03.08.2021   JP 2021127638**

(71) Applicant: **Kikkoman Corporation
Noda-shi, Chiba 278-8601 (JP)**

(72) Inventors:
• **SUZUKI Ryota
  Noda-shi, Chiba 278-8601 (JP)**
• **ICHIYANAGI Atsushi
  Noda-shi, Chiba 278-8601 (JP)**

(74) Representative: **J A Kemp LLP
80 Turnmill Street
London EC1M 5QU (GB)**

Remarks:
The complete document including Reference
Table(s) and the Sequence Listing(s) can be
downloaded from the EPO website

(54) **NOVEL FLAVIN-DEPENDENT LACTATE DEHYDROGENASE, AND METHOD FOR IMPROVING STABILITY OF LACTATE DEHYDROGENASE**

(57)    The present invention relates to a novel flavin-dependent lactate dehydrogenase and a method for improving stability of lactate dehydrogenase.

EP 4 382 608 A1

**Description**

TECHNICAL FIELD

The present invention relates to a novel flavin-dependent lactate dehydrogenase (hereinafter referred to as LDH), a nucleic acid encoding the same, a host cell having the nucleic acid, a method for producing LDH including culturing the host cell, and a method for improving stability of lactate dehydrogenase.

BACKGROUND ART

Lactic acid concentration in blood and lactic acid concentration in sweat are known as markers that reflect fatigue and physical condition. Lactic acid is a major metabolite and is recognized to be important not only for an index of health management but also health evaluation including in critical ill and/or surgical patients, and lactate levels in body fluids can be an index for various pathological conditions such as circulatory failure, liver disorders, etc. Monitoring of lactate can be used to detect sepsis, hypoxia and the presence of cancerous tissue (Non-Patent Document 1).

In addition, as an enzyme that uses lactic acid as a substrate, FMN-dependent lactate dehydrogenase (hereinafter referred to as FMN-LDH) is known. Saccharomyces cerevisiae-derived FMN-LDH (Non-Patent Document 2) which has heretofore been known has a problem in stability.

PRIOR ART DOCUMENT

PATENT DOCUMENT

Patent Document 1: JP 2018-519507A

NON-PATENT DOCUMENT

Non-Patent Document 1: The Japanese Guidelines for the Management of Sepsis 2016, PNAS September 15, 2015, 112 (37), 11642-11647
Non-Patent Document 2: Methods Enzymol., 53, 238-56, 1978

SUMMARY OF THE INVENTION

PROBLEMS TO BE SOLVED BY THE INVENTION

As enzymes that use lactic acid as a substrate, lactate oxidase (hereinafter referred to as LOD), NAD-dependent lactate dehydrogenase (hereinafter referred to as NAD-LDH) that uses nicotinamide dinucleotide (NAD) as a coenzyme, and FMN-LDH that uses flavin mononucleotide (FMN) as a coenzyme have been known.

LOD has a problem of producing hydrogen peroxide as a reaction product. In particular, there is a concern that it is undesirable to employ a sensor equipped with an enzyme that can continuously produce hydrogen peroxide, which is a type of reactive oxygen species and a causative substance of oxidative stress, when the lactic acid sensor is used in close contact with the skin or, in some cases, is intended to be implanted subcutaneously. Further, hydrogen peroxide generated by the action of LOD has an adverse effect on the stabilization of LOD itself, and as a means of avoiding a decrease in LOD activity caused by this, a lactic acid sensor in which catalase is coexistent for the purpose of scavenging hydrogen peroxide has been also proposed (Patent Document 1).

NAD-LDH catalyzes an enzymatic reaction that does not produce hydrogen peroxide, so that the problem of forming hydrogen peroxide does not arise. However, in the enzymatic reaction system using NAD-LDH, lactic acid and pyruvic acid react reversibly, so there is a problem that accurate measurement values cannot be obtained for quantitative applications, whereby it is difficult to employ it as a sensor.

FMN-LDH also catalyzes enzymatic reactions that do not produce hydrogen peroxide so the problem of forming hydrogen peroxide does not arise. In addition, there is no problem with reversible reaction, and thus, among the above-mentioned three enzymes, it can be considered to be the most promising as a practical enzyme for the purpose of monitoring lactic acid.

However, Saccharomyces cerevisiae-derived FMN-LDH (Non-Patent Document 2) which has been known as of today has a problem in stability. An object of the present invention is to search for a novel flavin-dependent LDH excellent in stability and to provide a method for improving stability of LDH.

MEANS TO SOLVE THE PROBLEMS

The present inventors searched for a novel flavin-dependent LDH excellent in stability in view of the above-mentioned problems, discovered a mutant LDH improved in stability whereby they have completed the present invention.

The present invention includes the following embodiments.

(1) A lactate dehydrogenase which comprises an amino acid sequence selected from the following (i) to (iii):

(i) an amino acid sequence represented by SEQ ID NO: 3,
(ii) an amino acid sequence having 70% or more identify with the amino acid sequence represented by SEQ ID NO: 3, or
(iii) when an alignment with the amino acid sequence represented by SEQ ID NO: 3 is formed, an amino acid sequence having 70% or more identify with the amino acid sequence represented by SEQ ID NO: 3 in the region from positions 110 to 502 of SEQ ID NO: 3, and

contains deletion of an N-terminus, and/or mutation(s) at one or more positions selected from the group consisting of position 54, position 156, position 349 and position 428.
(2) The lactate dehydrogenase described in the above-mentioned (1), wherein the deletion of the N-terminus is the positions 2 to 3, positions 2 to 4, positions 2 to 5, positions 2 to 6, positions 2 to 7, positions 2 to 75, positions 2 to 83, or positions 2 to 91.
(3) The lactate dehydrogenase described in the above-mentioned (1) or (2), wherein the mutation at the position 54 is A54C.
(4) The lactate dehydrogenase described in any of the above-mentioned (1) to (3), wherein the mutation at the position 156 is Y156F.
(5) The lactate dehydrogenase described in any of the above-mentioned (1) to (4), wherein the mutation at the position 349 is Y349F.
(6) The lactate dehydrogenase described in any of the above-mentioned (1) to (5), wherein the mutation at the position 428 is F428L.
(7) A nucleic acid which encodes the lactate dehydrogenase described in any of the above-mentioned (1) to (6).
(8) A host cell which has the nucleic acid described in the above-mentioned (7).
(9) A method for producing lactate dehydrogenase, which comprises culturing the host cell described in the above-mentioned (8).
(10) A method for improving stability of lactate dehydrogenase, which comprises the steps of deleting an N-terminus and/or mutating at one or more positions selected from the group consisting of position 54, position 156, position 349 and position 428 of the lactate dehydrogenase having an amino acid sequence selected from the following (i) to (iii):

(i) the amino acid sequence represented by SEQ ID NO: 3,
(ii) an amino acid sequence having 70% or more identify with the amino acid sequence represented by SEQ ID NO: 3, or
(iii) when an alignment with the amino acid sequence represented by SEQ ID NO: 3 is formed, an amino acid sequence having 70% or more identify with the amino acid sequence represented by SEQ ID NO: 3 in the region from positions 110 to 502 of SEQ ID NO: 3.

EFFECTS OF THE INVENTION

According to the present invention, a novel flavin-dependent LDH excellent in stability, a nucleic acid encoding the same, a host cell having the nucleic acid, a method for producing LDH including culturing the host cell, and a method for improving stability of LDH can be provided.

BRIEF DESCRIPTION OF THE DRAWING

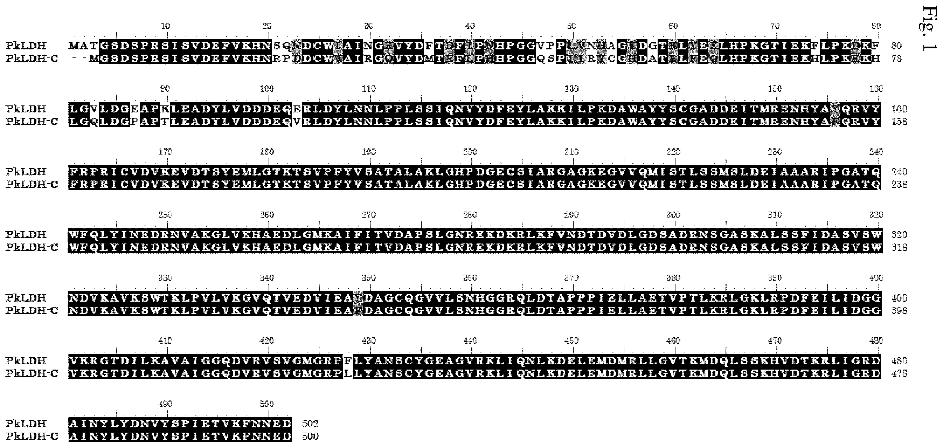

Fig. 1 is a drawing showing an alignment of the amino acid sequences of SEQ ID NO: 3 and SEQ ID NO: 5.

EMBODIMENTS TO CARRY OUT THE INVENTION

Hereinafter, embodiments of the present invention will be explained specifically. Incidentally, the following embodiments are examples of embodying the present invention, and do not limit the present invention within the range.

(LDH to which the present disclosure can be applied)

LDH referred to the present invention is an enzyme that catalyzes the reaction of oxidizing the hydroxyl group of lactic acid to produce pyruvic acid in the presence of an electron acceptor, which is similar to known wild type or mutant type LDH. Incidentally, unless otherwise specifically mentioned, the substrate of LDH is lactic acid, which may be provided as a mixture of L-form and D-form, for example, a racemate, or may be provided as L-form.

The LDH of the present invention may be LDH having an amino acid sequence selected from (i) an amino acid sequence represented by SEQ ID NO: 3, (ii) an amino acid sequence having high identity with the amino acid sequence represented by SEQ ID NO: 3, for example, 70% or more, more preferably 75% or more, further preferably 80% or more, further preferably 85% or more, further preferably 90% or more, 91% or more, 92% or more, 93% or more, 94% or more, further preferably 95% or more, 96% or more, 97% or more, 98% or more, and most preferably 99% or more identity, or (iii) when an alignment with the amino acid sequence represented by SEQ ID NO: 3 is formed, an amino acid sequence having high identity with the amino acid sequence represented by SEQ ID NO: 3 in the region from positions 110 to 502 of SEQ ID NO: 3, for example, 70% or more, more preferably 75% or more, further preferably 80% or more, further preferably 85% or more, further preferably 90% or more, 91% or more, 92% or more, 93% or more, 94% or more, further preferably 95% or more, most preferably 96% or more, 97% or more, 98% or more, and 99% or more identity, and containing deletion of an N-terminus, and/or mutation(s) at one or more positions selected from the group consisting of position 54, position 156, position 349 and position 428. More preferably, the LDH of the present invention has an N-terminus in which it is deleted at positions 2 to 3, positions 2 to 4, positions 2 to 5, positions 2 to 6, positions 2 to 7, positions 2 to 75, positions 2 to 83, or positions 2 to 91 in SEQ ID NO: 3. Also, more preferably, the mutation at position 54 in the LDH having an amino acid sequence represented by SEQ ID NO: 3 of the present invention is A54C, the mutation at position 156 is Y156F, the mutation at position 349 is Y349F, and the mutation at position 428 is F428L.

As a sequence having the amino acid sequence which has high identity with SEQ ID NO: 3 may be those having, for example, the amino acid sequence represented by SEQ ID NO: 5.

The LDH of the present invention may be sufficient with LDH as long as it has an amino acid sequence represented by SEQ ID NO: 5 or has high identity of, for example, 70% or more, more preferably 75% or more, further preferably 80% or more, further preferably 85% or more, further preferably 90% or more, 91% or more, 92% or more, 93% or more, 94% or more, further preferably 95% or more, 96% or more, 97% or more, 98% or more, and most preferably 99% or more, and contains deletion of an N-terminus, and/or mutation(s) at position 52 of SEQ ID NO: 5. More preferably, the LDH of the present invention has an N-terminus in which it is deleted at positions 2 to 3, positions 2 to 4, positions 2 to 5, positions 2 to 6, positions 2 to 7, positions 2 to 73, positions 2 to 81, or positions 2 to 89 in SEQ ID NO: 5. Also, more preferably, the mutation at position 52 (corresponding to position 54 of SEQ ID NO: 3 in alignment with SEQ ID NO: 3) in LDH having the amino acid sequence represented by SEQ ID NO: 5 of the present invention is S52C.

The LDH of the present invention exhibits, as mentioned later, LDH activity only in the amino acid sequence from positions 110 to 502 of the amino acid sequence represented by SEQ ID NO: 3. Accordingly, it can be understood that the amino acid sequence from positions 110 to 502 of the amino acid sequence represented by SEQ ID NO: 3 is a particularly important region for the function (activity) of LDH. The LDH of the present invention may be LDH having an amino acid sequence which has identity of 70% or more, 75% or more, 80% or more, 85% or more, 90% or more, 91% or more, 92% or more, 93% or more, 94% or more, more preferably 95% or more, 96% or more, 97% or more, 98% or more, and 99% or more with the amino acid sequence represented by SEQ ID NO: 3, when an alignment with the amino acid sequence represented by SEQ ID NO: 3 is formed, in the particularly important region (the region corresponding to positions 110 to 502 of SEQ ID NO: 3) for the function (activity) of LDH.

(About identity with amino acid sequence)

Identity of the amino acid sequence can be calculated by a program such as maximum matching or search homology, etc., of GENETYX Ver.11 (manufactured by Genetics) or a program such as maximum matching or multiple alignment, etc., of DNASIS Pro (manufactured by Hitachi Solutions).

(About corresponding positions in amino acid sequence)

In order to calculate amino acid sequence identity, by creating an alignment between the LDH represented by SEQ ID NO: 3 and another LDH, a location can be determined the position corresponding to a particular position of the LDH of SEQ ID NO: 3 in another LDH. For example, according to Fig. 1 (alignment of SEQ ID NO: 3 and SEQ ID NO: 5), position 5, position 6, position 7, position 54, position 75, position 83, position 91, position 110, position 156, position 349, position 428 and position 502 of SEQ ID NO: 3 correspond to position 3, position 4, position 5, position 52, position 73, position 81, position 89, position 108, position 154, position 347, position 426 and position 500 of SEQ ID NO: 5, respectively. Alignment of the amino acid sequence can be carried out using, for example, Blosum62 using CLUSTALW as an algorithm.

In one embodiment of the present invention, the present invention is a nucleic acid encoding the above-mentioned LDH.

in one embodiment of the present invention, the present invention is a host cell having the above-mentioned nucleic acid. As the host cell, it is not particularly limited as long as it is a conventional cell used in the field of the art. For example, there may be used *Escherichia coli* (*Escherichia coli*) K-12 strain and its derived strain (for example, JM109 strain), *Escherichia coli* B strain and its derived strain (for example, BL21 strain), *Bacillus subtilis* (for example, *Bacillus subtilis*, etc.), lactic acid bacteria (for example, *Lactococcus lactis,* etc.), other bacteria (*Corynebacterium glutamicum, Brevibacillus choshinensis,* etc.), yeast (for example, *Saccharomyces cerevisiae, Pichia pastoris, Schizosaccharomyces pombe,* etc.), filamentous fungi (for example, *Aspergillus oryzae, A. sojae, A. niger,* etc.), etc.

In one embodiment of the present invention, the present invention is a method for producing LDH, which comprises culturing the above-mentioned host cell.

In one embodiment of the present invention, the present invention is a method for improving stability of LDH which comprises, in an amino acid sequence represented by SEQ ID NO: 3 or an amino acid sequence having high identity thereto, for example, identity of 70% or more, more preferably 75% or more, further preferably 80% or more, further preferably 85% or more, further preferably 90% or more, 91% or more, 92% or more, 93% or more, 94% or more, further preferably 95% or more, 96% or more, 97% or more, 98% or more, and most preferably 99% or more, a step of deleting an N-terminus, and/or mutating one or more positions selected from the group consisting of position 54, position 156, position 349 and position 428. According to the present invention, LDH improved in stability can be produced.

In one embodiment of the present invention, the present invention is a method for improving stability of LDH which comprises, in an amino acid sequence represented by SEQ ID NO: 5 or an amino acid sequence having high identity thereto, for example, identity of 70% or more, more preferably 75% or more, further preferably 80% or more, further preferably 85% or more, further preferably 90% or more, 91% or more, 92% or more, 93% or more, 94% or more, further preferably 95% or more, 96% or more, 97% or more, 98% or more, and most preferably 99% or more, a step of deleting an N-terminus, and/or mutating position at 52. According to the present invention, LDH improved in stability can be produced.

In one embodiment of the present invention, the present invention is a method for improving stability of LDH which comprises, when an alignment with the amino acid sequence represented by SEQ ID NO: 3 is formed, in the particularly important region (the region corresponding to positions 110 to 502 of SEQ ID NO: 3) for the function (activity) of LDH, in an amino acid sequence represented by SEQ ID NO: 3 or an amino acid sequence having high identity thereto, for example, 70% or more, more preferably 75% or more, further preferably 80% or more, further preferably 85% or more, further preferably 90% or more, 91% or more, 92% or more, 93% or more, 94% or more, further preferably 95% or more, 96% or more, 97% or more, 98% or more, or most preferably 99% or more, a step of deleting an N-terminus, and/or mutating one or more positions selected from the group consisting of position 54, position 156, position 349 and position 428. According to the present invention, LDH improved in stability can be produced.

Activity of the LDH can be measured using this principle of action, for example, using the following measurement system which uses phenazine methosulfate (PMS) and 2,6-dichloroindophenol (DCIP) as electron acceptors.

(Reaction 1)     L-lactic acid + PMS (oxidized form) $\rightarrow$ Pyruvic acid + PMS (reduced form)

(Reaction 2)     PMS (reduced form) + DCIP (oxidized form) $\rightarrow$ PMS (oxidized form) + DCIP (reduced form)

Specifically, first, in (Reaction 1), accompanied with oxidation of L-lactic acid, PMS (reduced form) is formed. Then, by (Reaction 2) proceeding subsequently, accompanied with oxidation of PMS (reduced form), DCIP is reduced. The degree of disappearance of this "DCIP (oxidized form)" is detected as an amount of change in absorbance at a wavelength of 520 nm, and enzyme activity can be obtained based on this amount of change.

Specifically, activity of LDH can be measured according to the following procedure. 170 $\mu$L of 1M potassium phosphate buffer (pH 7.5), 300 $\mu$L of 50 mM DL-lactic acid solution, 250 $\mu$L of 1.8 mM DCIP solution and 680 $\mu$L of ultrapure water are mixed, and maintained at 37°C for 2 minutes or longer. Then, 50 $\mu$L of 30 mM PMS solution and 50 $\mu$L of an enzyme sample solution are added to start the reaction. Absorbance at the start of the reaction and absorbance with a lapse of time are measured, the amount of decrease in absorbance at 520 nm per minute ($\Delta A_{520}$) accompanied with the progress of the enzyme reaction is obtained, and LDH activity is calculated according to the following numerical formula 1. At this time, LDH activity is defined as 1 U, which is the amount of enzyme that reduces 1 $\mu$mol of DCIP per minute in the presence of L-lactic acid at a concentration of 10 mM at 37°C.

[Numerical formula 1]

$$\text{LDH activity}(U/mL)$$

$$=(-1)\times\frac{(\Delta Abs_{520}-\Delta Abs_{520,\,blank})\times1.5\times Df}{6.8\times0.05\times1.0}$$

$$=-4.41\times(\Delta Abs_{520}-\Delta Abs_{520,\,blank})\times Df$$

Incidentally, 1.5 in the formula represents a liquid amount (mL) of reaction reagent + enzyme reagent, 6.8 represents a millimolar molecular extinction coefficient ($mM^{-1}cm^{-1}$) of DCIP under the present activity measurement condition, 0.05 represents a liquid amount (mL) of an enzyme solution, 1.0 represents an optical path length (cm) of a cell, $\Delta A_{520,\,blank}$ represents a decreased amount in absorbance at 520 nm per minute when the reaction is started by adding 10 mM potassium phosphate buffer (pH 6.0) containing 0.15% (w/v) of bovine serum albumin in place of an enzyme sample solution, and Df represents a number of diluted fold.

(Evaluation method of thermal stability of LDH)

Thermal stability of LDH can be evaluated by heating LDH at a predetermined temperature for only a predetermined time, and comparing the activity before and after heating. Specifically, a 150 mM potassium phosphate buffer (pH 7.5) containing 0.15% (w/v) bovine serum albumin at a final concentration which contains LDH is allowed to stand on ice, and after heated to a predetermined temperature (for example, it can be 45°C, 50°C, 55°C or 60°C) for 15 minutes, respectively, LDH activity is measured. By setting the LDH activity of the LDH solution allowed to stand on ice without subjecting to heat treatment to be 100, activity of the LDH solution after heating is calculated, whereby a residual activity rate (%) can be measured.

(Improvement of stability of LDH)

The "improvement of stability" referred to in this disclosure include not only "improvement of thermal stability in a solution state", but also "improvement of thermal stability in a dry state", or "improvement of thermal stability in a drying step", or "improvement of storage stability (long-term stability) in a solution state" or "improvement of storage stability (long-term stability) in a dry state".

That is, the "improvement of stability" referred to in this disclosure means that a residual activity rate (%) of LDH which is a value in which after a composition containing LDH is, in the coexistence of a specific stabilizer, subjected to heat treatment for a certain period of time under a certain temperature condition, or after long-term storage, is increased as compared with the state before introducing the above-mentioned mutation.

Specifically, the residual activity rate (%) can be obtained by measuring the LDH activity value (a) of the solution before heat treatment or before long-term storage, and the LDH activity value (b) after heat treatment or after long-term storage are each measured, and calculating $((b)/(a)\times100)$. When the residual activity rate (%) of LDH after introduction of mutation which is after heat treatment or long-term storage is calculated, which is defined to be A, and when the residual activity rate (%) calculated by subjecting LDH before introduction of mutation which is a subject of comparison to the same treatment, which is defined to be B, and if A/B> 1, it is evaluated that the stability of LDH has improved.

Hereinafter, the present disclosure will be explained more specifically with reference to Examples. Provided that the technical scope of the present disclosure is not limited in any way by these examples.

EXAMPLES

1. Preparation of plasmids for expressing LDH

Preparation of various kinds of recombinant plasmids

578 amino acids represented by SEQ ID NO: 1 which is an amino acid sequence of *Pichia kudriavzevii*-derived LDH (PkLDH) and 506 amino acids of *Saccharomyces cerevisiae*-derived LDH (ScLDH) represented by SEQ ID NO: 2 are compared, and 1509 bp gene (containing stop codon TAA) represented by SEQ ID NO: 4, which encodes the 502 amino acids represented by SEQ ID NO: 3 from which positions 2 to 77 of SEQ ID NO: 1 have been deleted, was obtained as double-stranded DNA by PCR of a gene fragment which is a conventional method. Similarly, 1503 bp gene (containing stop codon TAA) represented by SEQ ID NO: 6, which encodes the 500 amino acids represented by SEQ ID NO: 5, was obtained as double-stranded DNA by PCR of a gene fragment which is a conventional method. These genes were inserted into the multiple cloning site of plasmid pKK223-3 by a conventional method to obtain a recombinant plasmid pKK223-3-PkLDH.

2. Preparation of plasmids for expressing mutant PkLDH

Using the obtained wild type (SEQ ID NO: 3) or plasmid pKK223-3-PkLDH for expressing mutant PkLDH as a template, and using synthetic oligonucleotides of SEQ ID NO: 7 to 27, and KOD-One PCR Master Mix (manufactured by Toyobo Co., Ltd.), PCR was carried out under the following conditions. The primer set used to prepare the plasmid for expressing mutant PkLDH is summarized in Table 1. That is, 25 $\mu$L of KOD-One PCR Master Mix, 50 ng of pKK223-3-PkLDH, and 15 pmol each of the above-mentioned synthetic oligonucleotides were added, and the total volume was made up to 50 $\mu$L with sterilized water. The prepared reaction solution was incubated at 94°C for 2 minutes using a thermal cycler (manufactured by Bio-Rad), subsequently, the cycle of "98°C for 10 sec" - "55°C for 5 sec" - "68°C for 40 sec" was repeated 7 to 15 times. The DNA thus obtained was treated with the restriction enzyme DpnI (available from NEW ENGLAND BIOLABS) to cleave the remaining template DNA, and when point mutation is to be introduced, *E. coli* JM109 was transformed using the reaction solution as such, and spread on LB-100 $\mu$g/mL ampicillin (hereinafter referred to as Amp) agar medium. When a part of the amino acid sequence is to be deleted, to 2.0 $\mu$L of DpnI-treated PCR product were added 5.0 $\mu$L of Ligation high Ver. 2 (manufactured by Toyobo Co., Ltd.), 1.0 $\mu$L of 5 U/$\mu$L T4 polyucleotide Kinase (manufactured by Toyobo Co., Ltd.), and 7.0 $\mu$L of sterilized water, and the mixture was reacted at 16°C for 1 hour and the resulting material was used for transformation. The grown colonies were inoculated on 2.5 mL of an LB-Amp medium [1% (w/v) Bact™ Tryptone, 0.5% (w/v) peptone, 0.5% (w/v) NaCl, and 100 $\mu$g/mL Amp] and cultured with shaking at 37°C for 20 hours to obtain a cultured product. This cultured product was centrifuged at 15,000 rpm for 5 minutes to collect bacteria and obtain bacterial cells. Then, a recombinant plasmid was extracted from the bacterial cells using FastGene Plasmid Mini Kit (manufactured by Nippon Genetics Co., Ltd.) and purified to obtain DNA.

[Table 1]

| Mutant | Template plasmid | Used primer |
|---|---|---|
| △A2~L75 | Wild type PkLDH | SEQ ID NO: 11, 12 |
| △A2~V83 | Wild type PkLDH | SEQ ID NO: 11, 13 |
| △A2~L91 | Wild type PkLDH | SEQ ID NO: 11, 14 |
| △A2~N109 | Wild type PkLDH | SEQ ID NO: 11, 27 |
| F428L | Wild type PkLDH | SEQ ID NO: 21, 22 |
| F428L/△A2 | F428L | SEQ ID NO: 11, 15 |
| F428L/△A2~T3 | F428L | SEQ ID NO: 11, 16 |
| F428L/△A2~G4 | F428L | SEQ ID NO: 11, 17 |
| F428L/△A2~S5 | F428L | SEQ ID NO: 11, 18 |
| F428L/△A2~D6 | F428L | SEQ ID NO: 11, 19 |
| F428L/△A2~S7 | F428L | SEQ ID NO: 11, 20 |
| Y156F/F428L/△A2~T3 | F428L/△A2~T3 | SEQ ID NO: 23, 24 |
| Y156F/Y349F/F428L/△A2~T3 | Y156F/F428L/△A2~T3 | SEQ ID NO: 25, 26 |
| A54C/Y156F/Y349F/F428L/△A2~T 3 | Y156F/Y349F/F428L/△A2~T3 | SEQ ID NO: 7, 8 |
| PkLDH-C/S52C | PkLDH-C | SEQ ID NO: 9, 10 |

3. Preparation of various LDH crude enzyme solutions

*E. coli* JM109 or BL21 strain was used as LDH producing bacteria. First, transformed *E. coli* JM109 or BL21 was picked from a colony of the cultured strain on an LB plate medium (containing 100 $\mu$g/mL Amp) in advance with a toothpick, cultured with shaking at 30°C and 180 rpm for 20 hours in a small test tube in which 4 mL of an LB medium (containing 100 $\mu$g/mL Amp and 0.1 mM isopropyl-$\beta$-thiogalactopyranoside (hereinafter referred to as IPTG)) was charged.

After completion of the culture, the culture solution was centrifuged at 7,000 rpm and 4°C for 5 minutes and the supernatant was removed, and the bacterial cells were collected. Then, the obtained bacterial cells were suspended in 600 $\mu$L of 150 mM potassium phosphate buffer (pH 7.5).

The above-mentioned bacterial cells suspension was sonicated using an ultrasonic homogenizer US-150E (manufactured by NIHONSEIKI KAISHA LTD.) suspension until the suspension became translucent, and centrifuged at 15,000 rpm and 4°C for 5 minutes to recover the supernatant. Using this supernatant of bacterial cell crushed liquid (hereinafter referred to as crude enzyme), enzyme activity was measured.

4. Verification 1 of effect of improving stability of PkLDH by introducing various mutations

Using PkLDH crude enzyme obtained as mentioned above, in accordance with the evaluation method of the thermal stability of LDH mentioned above, by comparing residual activity rate (%) before and after introducing mutation of LDH, search of mutation which improves stability of PkLDH was carried out.

Thermal stability of LDH was evaluated by heating LDH at a predetermined temperature for only a predetermined time, and comparing the activity before and after heating. Specifically, a 150 mM potassium phosphate buffer (pH 7.5) containing 0.15% (w/v) bovine serum albumin as a final concentration which contains LDH was allowed to stand on ice, and after heating at 55°C for 15 minutes, respectively, LDH activity was measured. Also, the LDH activity of the LDH solution allowed to stand on ice without subjecting to heat treatment was measured, and the activity is set to be 100, activity of the LDH solution after heating was calculated, whereby a residual activity rate (%) was measured. Specifically, the residual activity rate (%) was calculated by measuring the LDH activity value (a) of the solution before heat treatment or before long-term storage, and the LDH activity value (b) after heat treatment or after long-term storage are each measured, and calculating ((b)/(a)×100).

The extent of the effect of improving stability of LDH by introducing various mutations was determined by measuring the residual activity rate (%) of LDH after introducing various mutations and comparing it with the residual activity rate (%) before introducing the mutation.

Specifically, when the residual activity rate of the crude enzyme to be compared was set to 1, the relative value of the residual activity rate of the enzyme after introducing mutation was calculated, and if this value was greater than 1, it was considered to have an effect of improving stability. For example, if the residual activity rate of the crude enzyme to be compared was 80% and the residual activity rate of the enzyme after mutation introduction was 85%, the relative value of the residual activity will be 1.06.

Activity of LDH was measured according to the following procedure. 170 μL of 1M potassium phosphate buffer (pH 7.5), 300 μL of 50 mM L-lactic acid solution 250 μL of 1.8 mM DCIP solution and 680 μL of ultrapure water were mixed, and maintained at 37°C for 2 minutes or longer. Then, 50 μL of 30 mM PMS solution and 50 μL of an enzyme sample solution were added to start the reaction. Absorbance at the start of the reaction and absorbance with a lapse of time were measured, the amount of decrease in absorbance at 520 nm per minute ($\Delta A_{520}$) accompanied with the progress of the enzyme reaction was obtained, and LDH activity was calculated according to the following numerical formula 1. At this time, LDH activity was defined as 1 U, which is the amount of enzyme that reduces 1 μmol of DCIP per minute in the presence of L-lactic acid at a concentration of 10 mM at 37°C.

[Numerical formula 2]

$$\text{LDH activity (U/mL)}$$

$$= (-1) \times \frac{(\Delta Abs_{520} - \Delta Abs_{520,\ blank}) \times 1.5 \times Df}{6.8 \times 0.05 \times 1.0}$$

$$= -4.41 \times (\Delta Abs_{520} - \Delta Abs_{520,\ blank}) \times Df$$

Incidentally, 1.5 in the formula represents a liquid amount (mL) of reaction reagent + enzyme reagent, 6.8 represents a millimolar molecular extinction coefficient ($mM^{-1}cm^{-1}$) of DCIP under the present activity measurement condition, 0.05 represents a liquid amount (mL) of an enzyme solution, 1.0 represents an optical path length (cm) of a cell, $\Delta A_{520,\ blank}$ represents a decreased amount in absorbance at 520 nm per minute when the reaction is started by adding 10 mM potassium phosphate buffer (pH 6.0) containing 0.15% (w/v) of bovine serum albumin in place of an enzyme sample solution, and Df represents a number of diluted fold.

In accordance with the above-mentioned evaluation method of thermal stability, using LDH solution (2 to 3 U/mL) containing 150 mM of potassium phosphate buffer and 0.15% (w/v) bovine serum albumin (BSA), the residual activity rate of LDH was calculated.

In Table 2, the relative values of the residual activity rate of LDH containing N-terminal deletion according to the present invention is shown with the residual activity rate of the wild type PkLDH (Comparative Example) represented by SEQ ID NO: 3 being 1. In addition, in Table 3, the relative values of the residual activity rate of LDH containing N-terminal deletion according to the present invention is further shown with the residual activity rate of LDH (Present invention 4) having F428L in the wild type PkLDH represented by SEQ ID NO: 3 being 1.

Focusing on the amino acids on the N-terminal side of PkLDH, as a result of intensive investigation, as shown in Tables 2 and 3, among the amino acid sequences of PkLDH represented by SEQ ID NO: 3, it became clear that thermal stability of PkLDH is improved by introducing deletion of positions 2 to 75 ($\Delta$A2 to L75), deletion of positions 2 to 83 ($\Delta$A2 to V83), deletion of positions 2 to 91 ($\Delta$A2 to L91) and, F428L mutation and deletion of positions 2 (F428L/$\Delta$A2), F428L and deletion of positions 2 to 3 (F428L/$\Delta$A2 to T3), F428L and deletion of positions 2 to 4 (F428L/$\Delta$A2 to G4), F428L and deletion of positions 2 to 5 (F428L/$\Delta$A2 to S5), F428L and deletion of positions 2 to 6 (F428L/$\Delta$A2 to D6), or F428L and deletion of positions 2 to 7 (F428L/$\Delta$A2 to S7) into PkLDH. In addition, with regard to the mutant ($\Delta$A2 to N109) in which positions 2 to 109 of PkLDH had been deleted, LDH activity could be detected whereas heat resistance has not been evaluated.

[Table 2]

|  | Mutation | Relative value |
| --- | --- | --- |
| Comparative Example | Wild type PkLDH | 1 |
| Present invention 1 | $\Delta$A2~L75 | 1.5 |
| Present invention 2 | $\Delta$A2~V83 | 1.5 |
| Present invention 3 | $\Delta$A2~L91 | 1.1 |

[Table 3]

|  | Mutation | Relative value |
| --- | --- | --- |
| Present invention 4 | F428L | 1 |
| Present invention 5 | F428L/$\Delta$A2 | 1.1 |
| Present invention 6 | F428L/$\Delta$A2~T3 | 1.1 |
| Present invention 7 | F428L/$\Delta$A2~G4 | 1.1 |
| Present invention 8 | F428L/$\Delta$A2~S5 | 1.1 |
| Present invention 9 | F428L/$\Delta$A2~D6 | 1.2 |
| Present invention 10 | F428L/$\Delta$A2~S7 | 1.2 |

5. Verification 2 of effect of improving stability of PkLDH due to accumulation of mutations

In Table 4, the relative values of the residual activity rate of LDH containing Y156F mutation, Y349F mutation and F428L mutation according to the present invention is further shown with the residual activity rate of LDH (Present invention 6) having F428L in the wild type PkLDH represented by SEQ ID NO: 3 and containing deletion of positions 2 to 3 being 1.

As a result of intensive search for mutations that further improve the stability of F428L/$\Delta$A2 to T3 obtained as mentioned above, as shown in Table 4, it became clear that the resulting materials had higher stability than the original one by introducing Y156F mutation, Y349F mutation and F428L mutation.

[Table 4]

|  | Mutation | Relative value |
| --- | --- | --- |
| Present invention 6 | F428L/$\Delta$A2~T3 | 1 |
| Present invention 11 | Y156F/F428L/$\Delta$A2~T3 | 1.1 |
| Present invention 12 | Y156F/Y349F/F428L/$\Delta$A2~T3 | 1.2 |

6. Verification 3 of effect of improving stability of PkLDH due to accumulation of mutations

In Table 5, the relative values of the residual activity rate of LDH which further contains A54C and the amino acid sequence (hereinafter referred to as PkLDH-C) represented by SEQ ID NO: 5 with the residual activity rate of LDH (Present invention 12) having Y156F, Y349F, F428L in the wild type PkLDH represented by SEQ ID NO: 3 and containing deletion of positions 2 to 3 being 1.

As a result of intensive search for mutations that further improve the stability of PkLDH/Y156F/Y349F/F428L/ΔA2 to T3 obtained as mentioned above, as shown in Table 5, it became clear that the material into which A54C mutation had been introduced, and LDH having the amino acid sequence represented by SEQ ID NO: 5 had higher stability than the original one.

For reference, the results of alignment of the amino acid sequence of SEQ ID NO: 3 and SEQ ID NO: 5 are shown in Fig. 1.

[Table 5]

|  | Mutation | Relative value |
|---|---|---|
| Present invention 12 | Y156F/Y349F/F428L/ΔA2~T3 | 1 |
| Present invention 13 | A54C/Y156F/Y349F/F428L/ΔA2~T3 | 1.1 |
| Present invention 14 | PkLDH-C | 1.1 |

Subsequently, as a result of intensive search for mutations that further improve stability of LDH, as shown in Table 6, it became clear that the material in which S52C mutation (corresponding to A54C mutation of SEQ ID NO: 3) was introduced into PkLDH-C had higher stability than the original one.

[Table 6 ]

|  | Mutation | Relative value |
|---|---|---|
| Present invention 14 | PkLDH-C | 1 |
| Present invention 15 | PkLDH-C/S52C | 1.1 |

SEQUENCE LISTING

FP4787PCT.XML

**Claims**

1. Lactate dehydrogenase which comprises an amino acid sequence selected from the following (i) to (iii):

   (i) an amino acid sequence represented by SEQ ID NO: 3,
   (ii) an amino acid sequence having 70% or more identify with the amino acid sequence represented by SEQ ID NO: 3, or
   (iii) when an alignment with the amino acid sequence represented by SEQ ID NO: 3 is formed, an amino acid sequence having 70% or more identify with the amino acid sequence represented by SEQ ID NO: 3 in a region from positions 110 to 502 of SEQ ID NO: 3, and

   containing deletion of an N-terminus, and/or mutation(s) at one or more positions selected from the group consisting of position 54, position 156, position 349 and position 428.

2. The lactate dehydrogenase according to Claim 1, wherein N-terminal deletion is deletion at positions 2 to 3, positions 2 to 4, positions 2 to 5, positions 2 to 6, positions 2 to 7, positions 2 to 75, positions 2 to 83 or positions 2 to 91.

3. The lactate dehydrogenase according to Claim 1 or 2, wherein the mutation at position 54 is A54C.

4. The lactate dehydrogenase according to any one of Claims 1 to 3, wherein the mutation at position 156 is Y156F.

5. The lactate dehydrogenase according to any one of Claims 1 to 4, wherein the mutation at position 349 is Y349F.

6. The lactate dehydrogenase according to any one of Claims 1 to 5, wherein the mutation at position 428 is F428L.

7. A nucleic acid which encodes the lactate dehydrogenase according to any one of Claims 1 to 6.

8. A host cell which has the nucleic acid according to Claim 7.

9. A method for producing lactate dehydrogenase which comprises culturing the host cell according to Claim 8.

10. A method for improving stability of lactate dehydrogenase, which comprises the steps of deleting an N-terminus and/or mutating at one or more positions selected from the group consisting of position 54, position 156, position 349 and position 428 of the lactate dehydrogenase having an amino acid sequence selected from the following (i) to (iii):

(i) the amino acid sequence represented by SEQ ID NO: 3,
(ii) an amino acid sequence having 70% or more identify with the amino acid sequence represented by SEQ ID NO: 3, or
(iii) when an alignment with the amino acid sequence represented by SEQ ID NO: 3 is formed, an amino acid sequence having 70% or more identify with the amino acid sequence represented by SEQ ID NO: 3 in the region from positions 110 to 502 of SEQ ID NO: 3.

Fig. 1

EP 4 382 608 A1

```
              10         20         30         40         50         60         70         80
PkLDH    MATGSDSPRSISVDEFVKHNSQNDCWIAINGKVYDFTDFIPNHPGGVPPLVNHAGYDGTKLYEKLHPKGTIEKFLPKDKF  80
PkLDH-C  --MGSDSPRSISVDEFVKHNRPDDCWVAIRGQVYDMTEFLPHHPGGQSPIIRYCGHDATELFEQLHPKGTIEKHLPKEKH  78

              90        100        110        120        130        140        150        160
PkLDH    LGVLDGEAPKLEADYLVDDDEQERLDYLNNLPPLSSIQNVYDFEYLAKKILPKDAWAYYSCGADDEITMRENHYAYQRVY  160
PkLDH-C  LGQLDGPAPTLEADYLVDDDEQVRLDYLNNLPPLSSIQNVYDFEYLAKKILPKDAWAYYSCGADDEITMRENHYAFQRVY  158

             170        180        190        200        210        220        230        240
PkLDH    FRPRICVDVKEVDTSYEMLGTKTSVPFYVSATALAKLGHPDGECSIARGAGKEGVVQMISTLSSMSLDEIAAARIPGATQ  240
PkLDH-C  FRPRICVDVKEVDTSYEMLGTKTSVPFYVSATALAKLGHPDGECSIARGAGKEGVVQMISTLSSMSLDEIAAARIPGATQ  238

             250        260        270        280        290        300        310        320
PkLDH    WFQLYINEDRNVAKGLVKHAEDLGMKAIFITVDAPSLGNREKDKRLKFVNDTDVDLGDSADRNSGASKALSSFIDASVSW  320
PkLDH-C  WFQLYINEDRNVAKGLVKHAEDLGMKAIFITVDAPSLGNREKDKRLKFVNDTDVDLGDSADRNSGASKALSSFIDASVSW  318

             330        340        350        360        370        380        390        400
PkLDH    NDVKAVKSWTKLPVLVKGVQTVEDVIEAYDAGCQGVVLSNHGGRQLDTAPPPIELLAETVPTLKRLGKLRPDFEILIDGG  400
PkLDH-C  NDVKAVKSWTKLPVLVKGVQTVEDVIEAFDAGCQGVVLSNHGGRQLDTAPPPIELLAETVPTLKRLGKLRPDFEILIDGG  398

             410        420        430        440        450        460        470        480
PkLDH    VKRGTDILKAVAIGGQDVRVSVGMGRPFLYANSCYGEAGVRKLIQNLKDELEMDMRLLGVTKMDQLSSKHVDTKRLIGRD  480
PkLDH-C  VKRGTDILKAVAIGGQDVRVSVGMGRPLLYANSCYGEAGVRKLIQNLKDELEMDMRLLGVTKMDQLSSKHVDTKRLIGRD  478

             490        500
PkLDH    AINYLYDNVYSPIETVKFNNED  502
PkLDH-C  AINYLYDNVYSPIETVKFNNED  500
```

## INTERNATIONAL SEARCH REPORT

| International application No. |
| --- |
| **PCT/JP2022/028959** |

### A. CLASSIFICATION OF SUBJECT MATTER

*C12N 15/53*(2006.01)i; *C12N 1/15*(2006.01)i; *C12N 1/19*(2006.01)i; *C12N 1/21*(2006.01)i; *C12N 5/10*(2006.01)i; *C12N 9/04*(2006.01)i; *C12P 21/02*(2006.01)i; *C12Q 1/32*(2006.01)i

FI:  C12N15/53; C12N1/15; C12N1/19; C12N1/21; C12N5/10; C12P21/02 Z; C12Q1/32; C12N9/04 F ZNA

According to International Patent Classification (IPC) or to both national classification and IPC

### B. FIELDS SEARCHED

Minimum documentation searched (classification system followed by classification symbols)

C12N15/53; C12N1/15; C12N1/19; C12N1/21; C12N5/10; C12N9/04; C12P21/02; C12Q1/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

### C. DOCUMENTS CONSIDERED TO BE RELEVANT

| Category* | Citation of document, with indication, where appropriate, of the relevant passages | Relevant to claim No. |
| --- | --- | --- |
| X | Uniprot [online], Accession No. A0A1Q2YEU1 (entry version 17), 02 June 2021, < https://rest.uniprot.org/unisave/A0A1Q2YEU1?format=txt&versions=17>, [retrieved on 29 September 2022], Definition: FMN hydroxy acid dehydrogenase domain-containing protein whole document | 1, 7-10 |
| A | | 2-6 |
| X | Uniprot [online], Accession No. W1QKE8 (entry version 41), 02 June 2021, < https://rest.uniprot.org/unisave/W1QKE8?format=txt&versions=41>, [retrieved on 29 September 2022], Definition: Cytochrome b2, mitochondrial whole document | 1, 7-10 |
| A | | 2-6 |
| Y | JP 2021-78427 A (AISIN SEIKI CO., LTD.) 27 May 2021 (2021-05-27) whole document in particular, claims, examples | 1-10 |
| Y | JP 2007-75032 A (ORIENTAL YEAST CO., LTD.) 29 March 2007 (2007-03-29) whole document in particular, claims, examples | 1-10 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **03 October 2022** | **11 October 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2022/028959**

### C.    DOCUMENTS CONSIDERED TO BE RELEVANT

| Category* | Citation of document, with indication, where appropriate, of the relevant passages | Relevant to claim No. |
|---|---|---|
| Y | CN 112813043 A (JIANGNAN UNIVERSITY) 18 May 2021 (2021-05-18)<br>whole document in particular, claims, examples | 1-10 |
| P, X | WO 2021/167011 A1 (KIKKOMAN CORP.) 26 August 2021 (2021-08-26)<br>examples | 1-10 |

Form PCT/ISA/210 (second sheet) (January 2015)

**INTERNATIONAL SEARCH REPORT**

International application No.

**PCT/JP2022/028959**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above shall be read as "in the form of ST.26".

Form PCT/ISA/210 (continuation of first sheet) (January 2015)

INTERNATIONAL SEARCH REPORT
Information on patent family members

International application No.

**PCT/JP2022/028959**

| Patent document cited in search report | | | Publication date (day/month/year) | Patent family member(s) | Publication date (day/month/year) |
|---|---|---|---|---|---|
| JP | 2021-78427 | A | 27 May 2021 | (Family: none) | |
| JP | 2007-75032 | A | 29 March 2007 | (Family: none) | |
| CN | 112813043 | A | 18 May 2021 | (Family: none) | |
| WO | 2021/167011 | A1 | 26 August 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018519507 A

**Non-patent literature cited in the description**

- The Japanese Guidelines for the Management of Sepsis 2016. *PNAS,* 15 September 2015, vol. 112 (37), 11642-11647

- *Methods Enzymol.,* 1978, vol. 53, 238-56